# EUROPEAN PATENT APPLICATION

(11) **EP 1 926 324 A1**
(43) Date of publication of application: **28.05.2008**
(21) Application number: 06024159.3
(22) Date of filing: 21.11.2006
(51) Int. Cl.: H04N 13/04

(54) **System and method for displaying images in an overlaying relationship**

(71) Applicant: Swiss Medical Technology GmbH, 9443 Widnau (CH)
(72) Inventor: The designation of the inventor has not yet been filed
(74) Representative: Zwicker, Jörk

(57) **Abstract**

A system and a method for displaying a first image of an object of interest from a first source and a second image of an object of interest from a second source in an overlaying relationship are described. The system (10) comprises a display unit (12) having two input channels for receiving and displaying the first image and the second image, and a control unit (14) configured to supply one input channel of the display unit (12) with the first image from a first source and the other input channel of the display unit (12) with the second image from a different second source, such that said first and second image can be displayed in an overlaying relationship by the display unit (12). Preferably, the display unit (12) is an auto-stereoscopic or a stereoscopic monitor.

## Description

### TECHNICAL FIELD OF THE INVENTION

The invention relates to a system and a method for displaying images and in particular for displaying a first and a second image in an overlaying relationship on the screen of a single display unit.

### BACKGROUND OF THE INVENTION

In the medical field it is becoming more and more widespread to employ imaging systems for assisting medical professionals in tasks such as surgeries, dental inspections and the like. Commonly, an object of interest, e.g. a patient or a part thereof, is imaged by a camera and the image obtained by the camera is displayed on a monitor, where it can be viewed by the medical professional. In addition to such real-time images of the object of interest it would be desirable to overlay such images provided, for instance by a surgical microscope, with additional data, such as a corresponding portion of an image of the object of interest taken prior to the surgery. Practically, this cannot be accomplished by means of conventional monitors, since these would have to be alternately switched between the two images to be overlaid which is extremely time consuming and bears the risk of overlooking important corresponding features of the two images to be overlaid. If alternatively according to a further conventional method for overlaying images the additional data is simply superposed, only data with reduced content information can be used.

### SUMMARY OF THE INVENTION

It is, thus, the object of the present invention to provide a system and a method for displaying a first and a second image in an overlaying relationship, wherein the information content of the images to be overlaid is substantially preserved.

This object is achieved by a system according to claim 1 of the present invention as well as a method according to claim 8 of the present invention.

Further preferred beneficial embodiments are defined in the sub-claims.

### BRIEF DESCRIPTION OF THE DRAWINGS

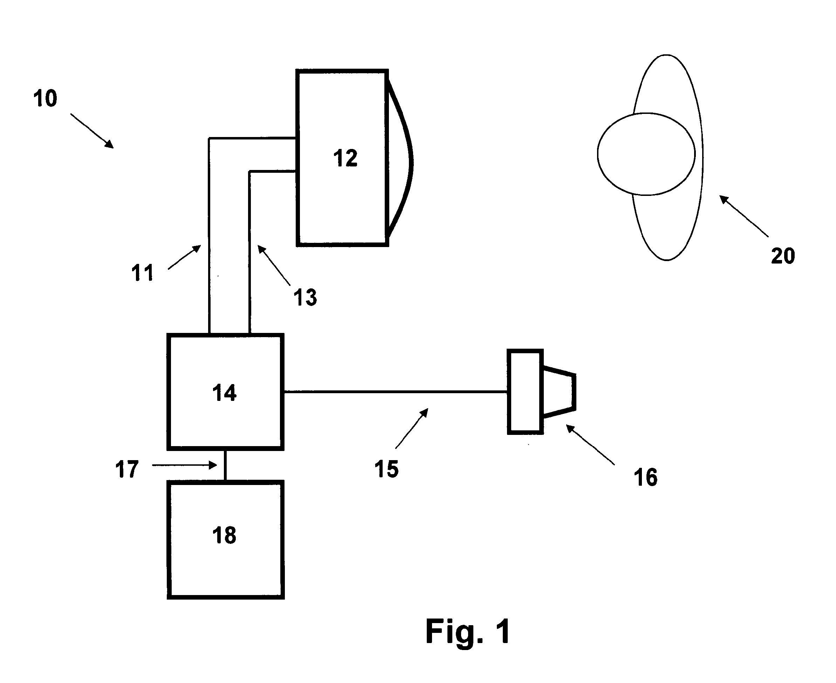

FIGURE 1 shows schematically a preferred embodiment of the system for displaying a first and a second image in an overlaying relationship according to the present invention.

### DETAILED DESCRIPTION OF PREFERRED EMBODIMENTS

A preferred embodiment of the system 10 for displaying a first and a second image in an overlaying relationship according to the present invention is shown in Figure 1. A display unit 12 with two input channels is connected via lines 11 and 13 to a control unit 14. According to a preferred embodiment the display unit 12 is an auto-stereoscopic monitor. As the person skilled in the art will recognize, auto-stereoscopic monitors can present a three-dimensional image to the viewer 20 without the need for the viewer 20 to use special glasses or other viewing aids. Alternatively, the display unit 12 preferably can be a stereoscopic monitor being viewed by the viewer 20 wearing active shutter glasses, polarized glasses or other suitable means well known to the person skilled in the art.

Auto-stereoscopic and stereoscopic monitors display a left eye image and a right eye image, which are transmitted to the brain of the viewer 20 for processing. In order to provide a stereoscopic effect several techniques have been developed to ensure that each eye sees the image it is supposed to see, i.e. the left eye of the viewer 20 sees the left eye image and the right eye sees the right eye image. When, for instance, a viewer is looking through synchronized shutter glasses at a stereoscopic monitor, the glasses "shutter" on and off, alternatively showing the left eye only the left eye image and the right eye only the right eye image. The shutter switches on and off so quickly that the viewer's brain "fuses" the two images into a single stereoscopic image such that a three-dimensional stereo image is perceived. As known to the person skilled in the art a similar effect can be provided in the case of auto-stereoscopic monitors by techniques known, for instance, as parallax barrier or lenticular plate.

According to the preferred embodiment of the system 10 for displaying a first and a second image in an overlaying relationship according to the present invention as shown in Figure 1 the control unit 14 is configured to supply one input channel of the display unit 12 e.g. via line 11 with a first image of an object of interest and the other input channel of the display unit 12 via line 13 with a second image of the object of interest from a different source than the first image. The display unit 12 is configured to display on the screen thereof the first image as a left eye image, i.e. an image to be seen by the left eye of the viewer 20, and the second image from a different source than the first image as a right eye image, i.e. an image to be seen by the right eye of the viewer 20, such that the viewer 20 will see the first and second images from different sources displayed in an overlaying relationship on the screen of the display unit 12. Preferably, the first image is provided in real time via line 15 by a camera 16, a microscope or the like. The second image form a different source than the first image preferably can be stored in a memory unit 18 and retrieved by the control unit 14 via line 17. Although the memory unit 18 is shown in Figure 1 to be external to the control unit 14, it is also contemplated that the memory unit can be part of the control unit 14.

Although only one camera 16 is shown in figure 1, also the second image of the object of interest could be provided in real time by a second separate imaging device (not shown), such as a camera, microscope or the like, being connected to the control unit 14. Alternatively, both images could be stored in the memory unit 18, such as a CT image as well as a MR image of the same object of interest. The person skilled in the art will appreciate that although in this case both images of the same object of interest are retrieved from the memory unit 18 these images are from different sources in the sense of the present application, namely from a device for performing CT and from a device for performing MR, respectively.

Preferably, the system 10 according to the present invention also comprises means for aligning the first and second image from different sources displayed by the display unit 12 on the screen thereof. As the person skilled in the art will appreciate this alignment could be provided e.g. by means of an appropriately configured software algorithm implemented within the display unit 12 or the control unit 14. Such a software algorithm would try to locate, preferably in real time, unique corresponding features within the first and the second image from different sources of the same object of interest to be overlaid. The software algorithm would preferably locate at least two of such features in order to be able to ensure the correct alignment of the first and the second image from different sources of the same object of interest.

By means of the system 10 according to the present invention the viewer 20 is able to "switch" almost instantaneously between three different views of an object of interest, namely (i) a first and a second image from different sources of the object of interest in an overlaying relationship, (ii) only the first image from a first source or (iii) only the second image from a second source. In case an auto-stereoscopic or stereoscopic monitor is used as a display unit the viewer can achieve such a switching between different views simply by closing/opening his left and right eyes, respectively.

As already mentioned above, the system 10 according to the present invention can be employed rather beneficially in the medical field. For instance, in the case of a dentist performing a dental treatment or a cosmetic surgeon performing a cosmetic surgery with the assistance of the system 10 according to the present invention it is contemplated that one channel of the display unit can be provided with a real-time image of the dental region or e.g. the facial region of a patient acquired by a camera or a microscope. The other channel of the display unit could be provided by an image of the dental region or the facial region of the patient prior and/or after the treatment, said image being stored within the memory unit. The person skilled in the art will appreciate that such a "post-treatment" image could be created by appropriately processing a "pre-treatment" image or other "pre-treatment" data of the dental region or the facial region of the patient with conventional software procedures. Obviously, the possibility of the dentist or the cosmetic surgeon in such a case to compare the current state of the treatment (i.e. the real time image) with the original state (i.e. the "pre-treatment" image") and/or the goal of the treatment (i.e. the "post-treatment" image) and to view these images separately or in an overlaying relationship greatly supports his task to provide for an appropriate and successful treatment.

The present invention as described in detail above is not limited to the particular devices, uses and methodology described as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step.

Several documents are cited throughout the text of this specification. Each of the documents cited herein (including all patents, patent applications, scientific publications, manufacturers specifications, instructions, etc.), whether *supra* or *infra,* are hereby incorporated by reference in their entirety. Nothing herein is to be construed as an admission that the invention is not entitled to antedate such disclosure by virtue of prior invention.

## Claims

1. A system (10) for displaying a first image of an object of interest from a first source and a second image of the object of interest from a second source in an overlaying relationship, comprising:
a display unit (12) having two input channels for receiving and displaying the first and the second image; and
a control unit (14) configured to supply one input channel of the display unit (12) with the first image and the other input channel of the display unit (12) with the second image from the second source, such that the first and the second image from different sources can be displayed in an overlaying relationship by the display unit (12).

2. The system according to claim 1, wherein the first source and/or the second source is a camera (16) connected to the control unit (14).

3. The system according to claim 1, wherein the first source and/or the second source is a microscope connected to the control unit (14).

4. The system according to any one of claims 1 to 3, wherein the first source and/or the second source is a memory unit (18) connected to the control unit (14).

5. The system according to any one of claims 1 to 4 further comprising means for aligning the first image and the second image on the display unit (12).

6. The system according to claim 5, wherein the means for aligning the first image and the second image comprise a software algorithm implemented in the control unit (14).

7. The system according to any one of claims 1 to 6, wherein the display unit (12) is a stereoscopic monitor or an auto-stereoscopic monitor.

8. Method of displaying a first image of an object of interest from a first source and a second image of the object of interest from a second source in an overlaying relationship, comprising the steps of:
providing the first image from the first source by means of a control unit (14) to a first input channel of a display unit (12),
providing the second image from the second source by means of the control unit (14) to the second input channel of the display unit (12), and
displaying the first image from the first source and the second image from the second source in an overlaying relationship on the screen of the display unit (12).

9. The method according to claim 8, wherein the first image is provided by a camera (16) or a microscope connected to the control unit (14).

10. The method according to claim 8 or 9, wherein the second image is retrieved from a memory unit (18) connected to the control unit (14).

11. The method according to claim 8, wherein the first and second image are aligned by the display unit (12) or the control unit (14) prior to being displayed.

12. The method according to any one of claims 8 to 11, wherein the display unit (12) is a stereoscopic monitor or an auto-stereoscopic monitor.
